# EUROPÄISCHE PATENTSCHRIFT

(11) **EP 0 476 474 B1**
(45) Veröffentlichungstag und Bekanntmachung des Hinweises auf die Patenterteilung: **28.12.1994**
(21) Anmeldenummer: 91115184.3
(22) Anmeldetag: 09.09.1991
(51) Int. Cl.: C07D 307/89

(54) **4-Amino-3-hydroxy-phthalid, sowie ein Verfahren zu seiner Herstellung**
4-Amino-3-hydroxy-phthalide and a method for its preparation
Amino-4 hydroxy-3 phtalide ainsi qu'un procédé pour sa préparation

(30) Priorität: 20.09.1990 DE 4029807
(43) Veröffentlichungstag der Anmeldung: 25.03.1992
(73) Patentinhaber: BAYER AG, 51368 Leverkusen (DE)
(72) Erfinder: Stoltefuss, Jürgen, Dipl.-Ing., W-5657 Haan (DE)

(56) Entgegenhaltungen:
- DE-A- 2 705 414
- US-A- 4 730 056
- CHEMICAL ABSTRACTS, Band 78, Nr. 1, 8. JUnner 1973, Columbus, Ohio, USAWATANABE, TOKUHIRO et al. "Indoles. III. New synthesis of 4-indolecarboxylicacid." Seite 339, Spalte 1, Zusammenfassung-Nr. 4 058h
- CHEMICAL ABSTRACTS, Band 80, Nr. 25, 24. Juni 1974, Columbus, Ohio, USA AOKI, KATSUMICHI et al. "Fungicidal compositions containing 4-chlorophthalide derivatives." Seite 84, Spalte 2, Zusammenfassung-Nr. 141 791x
- CHEMICAL ABSTRACTS, Band 90, Nr. 23, 4. Juni 1979, Columbus, Ohio, USA HOUBION,J. A. et al. "The synthesis of unamiguously substituted 3-hydroxyphtha- lides."Seite 640, Spalte 2, Zusammenfassung-Nr. 186 703t
- CHEMIAL ABSTRACTS, Band 104, Nr. 1, 6. JUnner 1986, Columbus, Ohio, USA SLOAN,KENNETH B. et al. "Further reactions of 3-hy- droxy-1(3H)-isobenzofuranone withamines." Seite 523, Spalte 1, Zusammenfassung-Nr. 5 724q

## Beschreibung

Die vorliegende Erfindung betrifft 4-Amino-3-hydroxy-phthalid, ein wichtiges Zwischenprodukt zur Synthese von 3-substituierten-5-chinolin-carbonsäuren sowie ein Verfahren zu seiner Herstellung.

Aus den Publikationen J. Org. Chem. 1988, 53, 223-224; 53, 1199-1202 und J. of Med. Chem., 1988, Vol, 31, Nr. 4,824-830 sind 3-Hydroxyphthalide bekannt, die im Phenylring, gegebenenfalls mehrfach, durch Chlor, Carboxy oder Methoxy substituiert sind.

Gegenstand der vorliegenden Erfindung ist die neue Verbindung 4-Amino-3-hydroxyphthalid der Formel (I). 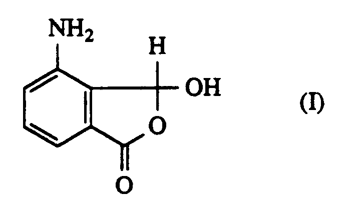
Die Erfindung betrifft weiterhin ein Verfahren zur Herstellung der Verbindung der Formel (I), dadurch gekennzeichnet, daß man
4-Nitro-3-hydroxyphthalid der Formel (II) 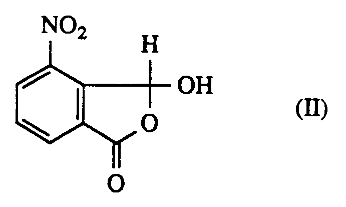
in inerten Lösemitteln, bevorzugt durch Hydrierung, in Anwesenheit eines Katalysators, reduziert.

Das Verfahren kann durch folgendes Formelschema erläutert werden: 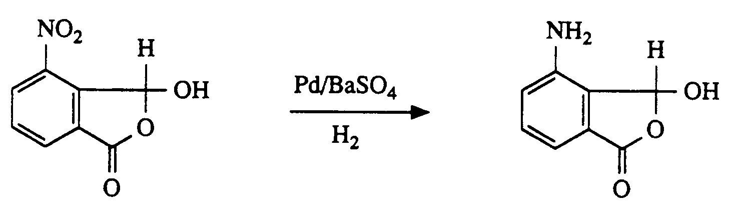
Als Lösemittel eignen sich für die Hydrierung alle organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Gylkoldimethylether, oder Diethylenglykoldimethylether oder Amide wie Hexamethylphosphorsäuretriamid oder Dimethylformamid, oder Essigsäure sowie Methylenchlorid, Tetrachlorkohlenstoff oder Toluol. Ebenso ist es möglich, Gemische der genannten Lösemittel zu verwenden. Bevorzugt sind Methanol, Ethanol, Propanol oder Tetrahydrofuran.

Die Hydrierung kann bei Normaldruck oder bei erhöhtem Druck, beispielsweise von 0,5 bis 5 bar, vorzugsweise bei Atmosphärendruck durchgeführt werden.

Die Reduktion erfolgt in allgemeinen in einem Temperaturbereich von 0°C bis 80°C, im Fall einer Hydrierung vorzugsweise bei Raumtemperatur.

Als Katalysatoren eignen sich Platin, Palladium, Palladium/Tierkohle, Palladium/Bariumsulfat oder Raney-Nickel. Besonders geeignet ist Palladium/Bariumsulfat.

Der Katalysator wird in einer Menge von 0,00001 bis 1 mol, bevorzugt von 0,001 bis 0,1 mol, bezogen auf 1 mol der Verbindung der Formel (II) eingesetzt.

Die Verbindungen der Formel (II) sind bekannt [vgl. T. Watanabe et al., Chem. Pharm. Bull, 20 (10), 2123-2127 (1972)].

Das vorstehende Herstellungsverfahren ist lediglich zur Verdeutlichung angegeben. Die Herstellung der erfindungsgemäße Verbindung der Formel (I) ist nicht auf dieses Verfahren beschränkt, sondern jede Modifikation dieses Verfahren, beispielsweise die Anwendung literaturbekannnter Nitro-Amino-Reduktionsmethoden, sind in gleicher Weise für die Herstellung der erfindungsgemäßen Verbindung anwendbar.

Die erfindungsgemäße Verbindung ist ein wertvolles Zwischenprodukt zur direkten Herstellung von teilweise bekannten oder neuen 3-substituierten Chinolin-5-carbonsäuren, die wiederum als Ausgangsstoffe für die entsprechenden 3-substituierten Chinolin-5-aldehyde dienen und somit in der 1,4-Dihydropyridin-Chemie von großer Bedeutung sind.

### Herstellungbeispiele

### Beispiel 1

### 4-Amino-3-hydroxyphthalid

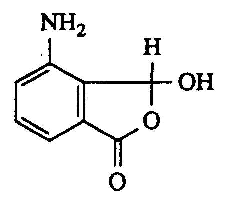

10 g 3-Hydroxy-4-nitro-phthalid werden in 100 ml Tetrahydrofuran gelöst und nach Zugabe von 1 g Palladium auf Bariumsulfat (5%) bei Atmosphärendruck und 20 - 25°C hydriert. Es wird vom Katalysator abfiltriert und eingeengt. Der Eindampfrückstand wird mit Ether verrührt und abgesaugt. Man erhält 5,8 g (68,5% der Theorie) einer farblosen Substanz vom Schmp. 280-285°C (Zers.).

### Beispiel 2

### 3-Phenyl-chinolin-5-carbonsäure

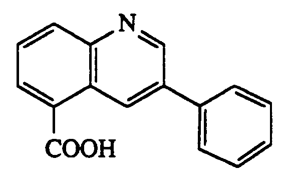

50 g (0,256 mol) 4-Hydroxy-4-nitro-phthalid werden in 380 ml Ethanol mit 5 g Palladium/Bariumsulfat (5%) bei 20°C und 3 bar hydriert. Es wird abgesaugt, das Filtrat wird mit 0,308 mol (38,7 ml) Phenylacetaldehyd versetzt. Es wird 4 Stunden gekocht, wobei die Chinolincarbonsäure ausfällt. Es wird abgekühlt, abgesaugt und mit Ethanol gewaschen. Man erhält 28,3 g (44,3% der Theorie) einer farblosen Verbindung vom Schmp. > 290°C.

## Patentansprüche (Patentansprüche für folgende(n) Vertragsstaat(en): AT, BE, CH, DE, DK, FR, GB, GR, IT, LI, LU, NL, SE)

1. 4-Amino-3-hydroxy-phthalid der Formel

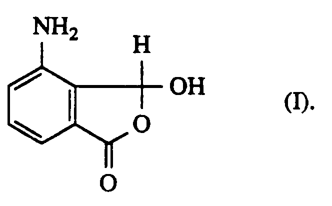

2. Verfahren zur Herstellung von 4-Amino-3-hydroxy-phthalid der Formel 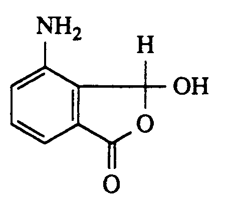 dadurch gekennzeichnet, daß man 4-Nitro-3-hydroxy-phthalid der Formel 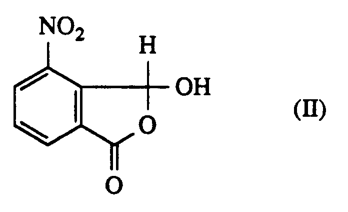 in inerten Lösemitteln reduziert.

3. Verfahren nach Anspruch 2, dadurch gekennzeichnet, daß man 4-Nitro-3-hydroxy-phthalid in Anwesenheit eines Katalysators hydriert.

4. Verfahren nach Anspruch 2, dadurch gekennzeichnet daß Katalysator Platin, Palladium, Palladium/Tierkohle, Palladium/Bariumsulfat oder Raney-Nickel einsetzt.

5. Verwendung von 4-Amino-3-hydroxy-phthalid der Formel 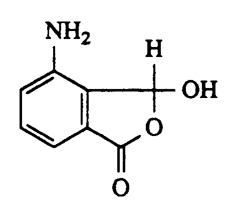 als Zwischenprodukt zur Herstellung von 3-substituierten Chinolin-5-carbonsäuren.

## Patentansprüche (Patentansprüche für folgende(n) Vertragsstaat(en): ES)

1. Verfahren zur Herstellung von 4-Amino-3-hydroxy-phthalid der Formel 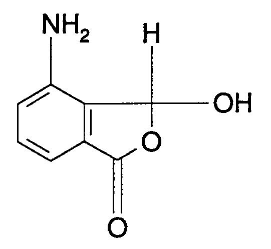 dadurch gekennzeichnet, daß man 4-Nitro-3-hydroxy-phthalid der Formel 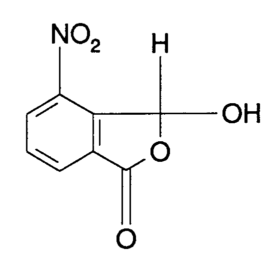 in inerten Lösemitteln reduziert.

2. Verfahren nach Anspruch 1, dadurch gekennzeichnet, daß man 4-Nitro-3-hydroxy-phthalid in Abwesenheit eines Katalysators hydriert.

3. Verfahren nach Anspruch 1, dadurch gekennzeichnet, daß man als Katalysator Platin, Palladium, Palladium/Tierkohle, Palladium/Bariumsulfat oder Raney-Nickel einsetzt.

4. Verwendung von 4-Amino-3-hydroxy-phthalid der Formel 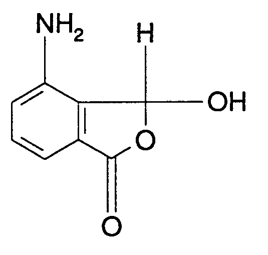 als Zwischenprodukt zur Herstellung von 3-substitutierten Chinolin-5-carbonsäuren.

## Claims (Claims for the following Contracting State(s): AT, BE, CH, DE, DK, FR, GB, GR, IT, LI, LU, NL, SE)

1. 4-Amino-3-hydroxyphthalide of the formula

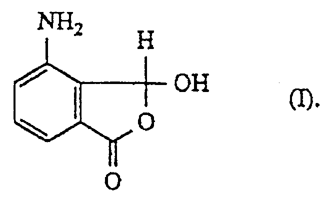

2. Process for the preparation of 4-amino-3-hydroxyphthalide of the formula 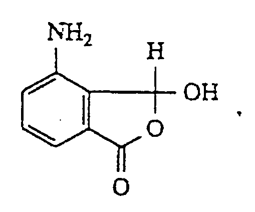 characterized in that 4-nitro-3-hydroxyphthalide of the formula 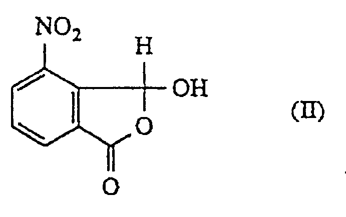 is reduced in inert solvents.

3. Process according to Claim 2, characterized in that 4-nitro-3-hydroxyphthalide is hydrogenated in the presence of a catalyst.

4. Process according to Claim 2, characterized in that the catalyst employed is platinum, palladium, palladium/animal charcoal, palladium/barium sulphate or Raney nickel.

5. Use of 4-amino-3-hydroxyphthalide of the formula 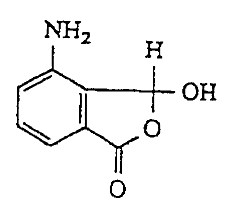 as intermediate for the preparation of 3-substituted 5-quinolinecarboxylic acids.

## Claims (Claims for the following Contracting State(s): ES)

1. Process for the preparation of 4-amino-3-hydroxyphthalide of the formula 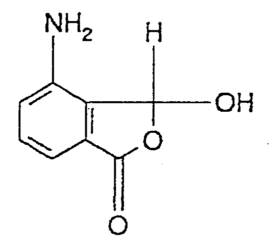 characterized in that 4-nitro-3-hydroxyphthalide of the formula 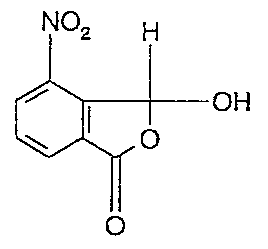 is reduced in inert solvents.

2. Process according to Claim 1, characterized in that 4-nitro-3-hydroxyphthalide is hydrogenated in the presence of a catalyst.

3. Process according to Claim 1, characterized in that the catalyst employed is platinum, palladium, palladium/animal charcoal, palladium/barium sulphate or Raney nickel.

4. Use of 4-amino-3-hydroxyphthalide of the formula 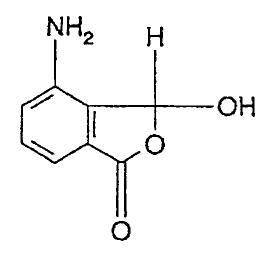 as intermediate for the preparation of 3-substituted 5-quinolinecarboxylic acids.

## Revendications (Revendications pour l'(les) Etat(s) contractant(s) suivant(s): AT, BE, CH, DE, DK, FR, GB, GR, IT, LI, LU, NL, SE)

1. Le 4-amino-3-hydroxyphtalide de formule

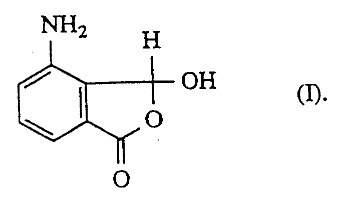

2. Procédé de production du 4-amino-3-hydroxyphtalide de formule 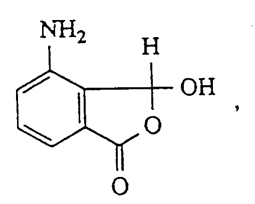 caractérisé en ce qu'on réduit le 4-nitro-3-hydroxyphtalide de formule 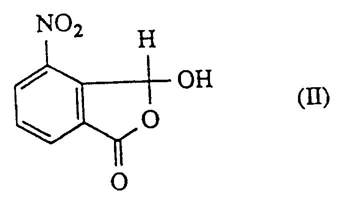 dans des solvants inertes.

3. Procédé suivant la revendication 2, caractérisé en ce qu'on hydrogène le 4-nitro-3-hydroxyphtalide en présence d'un catalyseur.

4. Procédé suivant la revendication 2, caractérisé en ce qu'on utilise comme catalyseur le platine, le palladium, le palladium sur du noir animal, le palladium sur du sulfate de baryum ou le nickel de Raney.

5. Utilisation du 4-amino-3-hydroxyphtalide de formule 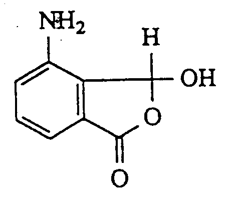 comme produit intermédiaire pour l'obtention d'acides quinoléine-5-carboxyliques substitués en position 3.

## Revendications (Revendications pour l'(les) Etat(s) contractant(s) suivant(s): ES)

1. Procédé de production du 4-amino-3-hydroxyphtalide de formule 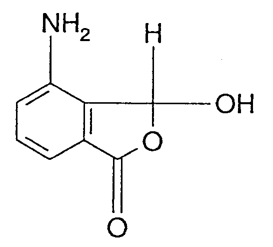 caractérisé en ce qu'on réduit le 4-nitro-3-hydroxyphtalide de formule 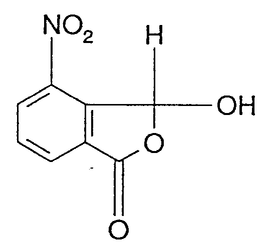 dans des solvants inertes.

2. Procédé suivant la revendication 1, caractérisé en ce qu'on hydrogène le 4-nitro-3-hydroxyphtalide en présence d'un catalyseur.

3. Procédé suivant la revendication 1, caractérisé en ce qu'on utilise comme catalyseur le platine, le palladium, le palladium sur du noir animal, le palladium sur du sulfate de baryum ou le nickel de Raney.

4. Utilisation du 4-amino-3-hydroxyphtalide de formule 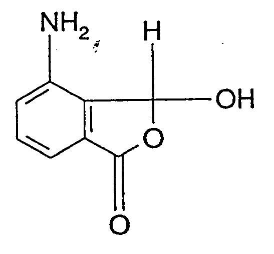 comme produit intermédiaire pour l'obtention d'acides quinoléine-5-carboxyliques substitués en position 3.
